# FASCICULE DE BREVET EUROPEEN

(11) **EP 1 181 926 B1**
(45) Date de publication et mention de la délivrance du brevet: **27.04.2005**
(21) Numéro de dépôt: 01401999.6
(22) Date de dépôt: 25.07.2001
(51) Int. Cl.: A61K 7/48

(54) **Composition cosmétique comprenant de la diosgénine et de la caféine**
Diosgenin und Coffein enthaltende Kosmetikzusammensetzung
Cosmetic compositiion comprising diosgenin and caffeine

(30) Priorité: 22.08.2000 FR 0010805
(43) Date de publication de la demande: 27.02.2002
(73) Titulaire: L'OREAL, 75008 Paris (FR)
(72) Inventeur: Picard-Lesboueyries, Elisabeth, 78140 Velizy (FR)
(74) Mandataire: Renard, Emmanuelle

(56) Documents cités:
- EP-A- 0 728 472
- WO-A-00/30603
- US-A- 5 770 223

## Description

La présente invention concerne une composition comprenant, dans un milieu physiologiquement acceptable, de la diosgénine ou un extrait de Dioscorée en contenant, et de la caféine.

Les sapogénines sont des composés résultant de l'hydrolyse acide des saponosides, qui sont eux-mêmes des hétérosides de poids moléculaire très élevé présents dans le règne végétal. Parmi les sapogénines, on citera en particulier : la diosgénine, l'hécogénine, la smilagénine, la sarsapogénine, la tigogénine, la yamogénine et la yuccagénine.

Ces composés ont en commun une structure stéroïde comprenant un nombre de substituants hydroxyle et/ou oxo variable et/ou un nombre variable de double liaisons. Ils sont connus comme précurseurs chimiques naturels d'hormones stéroïdiennes et décrits, à ce titre, comme constituants de choix de diverses préparations cosmétiques ou pharmaceutiques.

Une sapogénine préférée est la diosgénine, ou spirost-5-èn-3-beta-ol, qui peut être extraite de fenugrec ou de diverses Dioscorées, par exemple de racine d'igname sauvage.

La diosgénine a en particulier été décrite comme étant utilisable dans des compositions amincissantes, en raison de son aptitude à inhiber le stockage du matériel biochimique lipidique dans le tissu adipeux (FR-2 786 097).

Les documents FR 2 554 344 et FR 2 499 405 décrivent des compositions amincissantes comprenant une base xanthique, telle que la caféine et des extraits de plantes contenant des sapogénines.

Or, il est apparu à la Demanderesse que l'association à la diosgénine de caféine, elle-même bien connue dans des produits amincissants en tant qu'inhibiteur de phosphodiestérase et/ou de lipoprotéine lipase, pouvait permettre d'améliorer l'efficacité des compositions amincissantes contenant la diosgénine.

On connaît certes du brevet US-5,770,223 des compositions cosmétiques destinées à favoriser le renouvellement épidermique, qui associent des sapogénines de luzerne à divers actifs dont les bases xanthiques. Ces compositions sont destinées à combattre les effets du vieillissement de la peau et à stimuler la repousse des cheveux ou éviter leur chute. Ces sapogénines se différencient chimiquement de celles présentes, notamment, dans l'igname sauvage et l'agave. Les compositions obtenues ne sont pas décrites comme étant susceptibles d'avoir un effet amincissant.

La présente invention a donc pour objet une composition comprenant, dans un milieu physiologiquement acceptable, de la diosgénine ou un extrait de Dioscorée en contenant, et de la caféine.

La diosgénine peut être extraite des tubercules de Dioscorées par un procédé comprenant successivement : l'hydrolyse des hétérosides en milieu acide minéral (éventuellement après fermentation et séchage des tubercules) ; et la filtration de la fraction insoluble, qui est ensuite neutralisée, lavée et traitée par un solvant apolaire. D'autres procédés d'extraction sont cependant utilisables. La diosgénine est également disponible dans le commerce auprès de la société SIGMA sous la dénomination commerciale Diosgenin.

Par "extrait de Dioscorée", on entend notamment un extrait de rhizome d'igname sauvage (Dioscorea villosa ou Dioscorea mexicana ou Dioscorea opposita en particulier), qui contient de la diosgénine.

La diosgénine peut représenter de 0,001 à 10%, et de préférence de 0,05 à 5%, du poids total de la composition selon l'invention.

Cette composition renferme en outre, en plus de la diosgénine, de la caféine.

Cette base xanthique est connue comme inhibiteur de phosphodiestérase, qui est l'enzyme responsable de la dégradation de l'AMPc. En augmentant le taux intra-cellulaire d'AMPc, cette base xanthique favorise l'activité lipolytique et constitue donc un actif amincissant de premier ordre.

La caféine peut représenter de 0,01 à 10%, et de préférence de 0,1 à 7%, du poids total de la composition selon l'invention.

La composition selon l'invention peut se présenter sous toutes les formes galéniques normalement utilisées pour une application topique sur la peau, notamment sous forme d'une émulsion huile-dans-eau ou eau-dans-huile ou multiple, éventuellement gélifiée, d'une émulsion siliconée, d'une microémulsion ou nanoémulsion, d'un produit anhydre liquide, pâteux ou solide, d'une dispersion d'huile dans une phase aqueuse en présence de sphérules, ces sphérules pouvant être des nanoparticules polymériques telles que les nanosphères et les nanocapsules ou, mieux, des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse ou d'un gel. Elle peut éventuellement être appliquée sur la peau sous forme d'aérosol. Elle peut également se présenter sous forme solide, et par exemple sous forme de stick. Elle peut être utilisée comme produit de soin et/ou comme produit de maquillage de la peau.

De façon connue, la composition de l'invention peut contenir également les adjuvants habituels dans les domaines cosmétique et dermatologique, tels que les gélifiants hydrophiles ou lipophiles, les actifs hydrophiles ou lipophiles, les conservateurs, les antioxydants, les solvants, les parfums, les charges, les pigments, les filtres hydrophiles, les absorbeurs d'odeur et les matières colorantes. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse, dans la phase aqueuse, dans les vésicules lipidiques et/ou dans les nanoparticules.

Lorsque la composition selon l'invention est une émulsion, la proportion de la phase grasse peut aller de 5 à 80 % en poids, et de préférence de 5 à 50 % en poids par rapport au poids total de la composition.

Comme matières grasses utilisables dans l'invention, on peut utiliser les huiles minérales, les huiles d'origine animale, les huiles de synthèse, les huiles siliconées et les huiles fluorées. On peut aussi utiliser comme matières grasses des acides gras, des cires et des gommes et en particulier les gommes de silicone.

Les émulsionnants et les co-émulsionnants éventuellement utilisés dans la composition sous forme d'émulsion sont choisis parmi ceux classiquement utilisés dans le domaine considéré. Ces émulsionnants et coémulsionnants sont de préférence présents, dans la composition, en une proportion allant de 0,3 à 30 % en poids, et de préférence de 0,5 à 20 % en poids par rapport au poids total de la composition. Comme émulsionnants et coémulsionnants utilisables dans l'invention, il est particulièrement avantageux d'utiliser les esters d'acide gras et de polyol tels que le stéarate de PEG-100, le stéarate de PEG-50 et le stéarate de PEG-40 ; le tristéarate de sorbitane, les stéarates de sorbitane oxyéthylénés disponibles sous les dénominations commerciales Tween® 20 ou Tween® 60, par exemple ; et leurs mélanges.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras et la silice hydrophobe.

Comme actifs qui vont compléter l'action des actifs amincissants selon l'invention, on peut utiliser notamment *:*
- les actifs agissant sur la microcirculation (vasoprotecteurs ou vasodilatateurs), tels que les flavonoïdes, les extraits de Gingko biloba, les ruscogénines, les esculosides, l'escine extraite du marron d'Inde, les nicotinates, l'hespéridine méthyl chalcone, le ruscus ou petit houx, les huiles essentielles de lavande ou de romarin ;
- les actifs raffermissants et/ou anti-glycants (empêchant la fixation du sucre sur les fibres de collagène), tels que les extraits de Centella asiatica et de Siegesbeckia, qui stimulent la synthèse de collagène, le silicium, l'amadorine, la vitamine C et ses dérivés et le rétinol et ses dérivés ;
- et leurs mélanges.

En cas d'incompatibilité entre eux ou avec la diosgénine, les actifs indiqués ci-dessus et/ou la diosgénine peuvent être incorporés dans des sphérules, notamment des vésicules ioniques ou non-ioniques et/ou des nanoparticules (nanocapsules et/ou nanosphères), de manière à les isoler les uns des autres dans la composition.

Bien entendu, l'homme de l'art veillera à choisir le ou les éventuels composés à ajouter aux compositions selon l'invention, ainsi que leur concentration, de manière telle que les propriétés avantageuses attachées intrinsèquement aux compositions conformes à l'invention ne soient pas, ou substantiellement pas, altérées par l'addition envisagée.

L'invention concerne également l'utilisation d'une composition comprenant, dans un milieu physiologiquement acceptable, de la diosgénine ou un extrait de Dioscorée en contenant, et de la caféine, pour prévenir ou lutter contre la cellulite et/ou pour affiner la silhouette ou les contours du visage.

L'invention sera maintenant illustrée à l'aide des exemples non limitatifs suivants.

### EXEMPLES

### Exemple 1 : Crème amincissante pour le corps

| **Exemple 1 : Crème amincissante pour le corps** | | |
|---|---|---|
| A | Copolymère acrylates/C ₁₀₋₃₀ alkyl acrylate | 0,5% |
| | Eau | 12 % |
| | | |
| B | Hexyl décanol | 10 % |
| | Isononanoate d'isononyle | 10 % |
| | Diosgénine | 0,3% |
| | | |
| C | Acide salicylique | 2,5% |
| | Triéthanolamine | 4 % |
| | Eau | 20 % |
| | Glycérine | 6 % |
| | Caféine | 3 % |
| | | |
| D | Polyacrylamide et C₁₃₋₁₄ isoparaffin et laureth-7 | 0,5% |
| | | |
| E | Eau | qsp |
| | Alcool | 15 % |

La composition peut être préparée de la manière suivante. Le polymère de la phase A est dispersé dans l'eau sous agitation à 40°C. Les constituants de la phase B sont chauffés jusqu'à complète dissolution (70°C), puis la température est ramenée à 40°C. Les constituants de la phase C sont solubilisés à 50°C. La phase B est ensuite introduite dans la phase A sous agitation. Après vérification de l'absence de cristaux au microscope, les phases C, D puis E puis ajoutées au mélange précédemment obtenu.

### Exemple 2 : Crème amincissante pour le corps

| **Exemple 2 : Crème amincissante pour le corps** | | |
|---|---|---|
| A | Copolymère acrylates/C₁₀₋₃₀ alkyl acrylate | 0,5% |
| | Eau | 12 % |
| | | |
| B | Hexyl décanol | 10 % |
| | Isononanoate d'isononyle | 10 % |
| | Extrait de rhizome d'igname sauvage ( *Dioscorea villosa* ) titré à 3% en diosgénine | 0,3% |
| | | |
| C | Acide salicylique | 2,5% |
| | Triéthanolamine | 4 % |
| | Eau | 20 % |
| | Glycérine | 6 % |
| | Caféine | 3 % |
| | | |
| D | Polyacrylamide et C₁₃₋₁₄ isoparaffin et laureth-7 | 0,5% |
| | | |
| E | Eau | qsp |
| | Alcool | 15 % |

La composition ci-dessus peut être préparée comme décrit dans l'Exemple 1.

### Exemple 3 :Crème amincissante pour le visage

| | | |
|---|---|---|
| A | Eau déminéralisée | qsp |
| | Conservateurs | 0,25 % |
| | Carbomer | 0,4 % |
| | Glycérine | 3 % |
| | Caféine | 2 % |
| | | |
| B1 | Stéarate de sorbitane oxyéthyléné 20 OE (polysorbate 60) | 0,9 % |
| | | |
| B2 | Stéarate de PEG-100 et stéarate de glycéryle | 2,1 % |
| | Alcool cétylique | 2,6 % |
| | Isononanoate d'isononyle | 11,5 % |
| | Octyldodécanol | 15 % |
| | Diosgénine | 0,5 % |
| | Butyl hydroxytoluène | 0,1 % |
| | Filtre UVB | 1 % |
| | Conservateur | 0,15 % |
| | | |
| C | Eau déminéralisée | 2 % |
| | Triéthanolamine | 0,3 % |

La composition ci-dessus peut être préparée de la manière suivante. Les constituants des phases A, B1 et B2 sont portés à 70°C sous agitation. La phase C est préparée en mélangeant ses constituants à température ambiante. La phase B1 est ensuite ajoutée à la phase B2, puis le mélange des deux phases est versé dans la phase A sous agitation rapide. Le mélange obtenu est mélangé 10 minutes, avant introduction de la phase C sous agitation.

## Revendications

1. Composition comprenant, dans un milieu physiologiquement acceptable, de la diosgénine ou un extrait de Dioscorée en contenant, et de la caféine.

2. Composition selon la revendication 1, **caractérisée en ce que** ledit extrait de Dioscorée est un extrait de rhizome d'igname sauvage.

3. Composition selon la revendication1 ou 2, **caractérisée en ce que** la caféine représente de 0,01 à 10% du poids total de la composition.

4. Composition selon la revendication 3, **caractérisée en ce que** la caféine représente de 0,1 à 7% du poids total de la composition.

5. Utilisation d'une composition comprenant, dans un milieu physiologiquement acceptable, de la diosgénine ou un extrait de Dioscorée en contenant, et de la caféine, pour prévenir ou lutter contre la cellulite.

6. Utilisation d'une composition comprenant, dans un milieu physiologiquement acceptable, de la diosgénine ou un extrait de Dioscorée en contenant, et de la caféine, pour affiner la silhouette ou les contours du visage.

## Patentansprüche

1. Zusammensetzung, die in einem physiologisch akzeptablen Medium Diosgenin oder einen Diosgenin-haltigen Dioscorea-Extrakt und Coffein enthält.

2. Zusammensetzung nach Anspruch 1, **dadurch gekennzeichnet, dass** der Dioscorea-Extrakt ein Extrakt aus dem Rhizom von Wildem Yams ist.

3. Zusammensetzung nach Anspruch 1 oder 2, **dadurch gekennzeichnet, dass** das Coffein 0,01 bis 10 % des Gesamtgewichts der Zusammensetzung ausmacht.

4. Zusammensetzung nach Anspruch 3, **dadurch gekennzeichnet, dass** das Coffein 0,1 bis 7 % des Gesamtgewichts der Zusammensetzung ausmacht.

5. Verwendung einer Zusammensetzung, die in einem physiologisch akzeptablen Medium das Diosgenin oder einen Diosgenin-haltigen Dioscorea-Extrakt und Coffein enthält, um Cellulitis vorzubeugen oder zu bekämpfen.

6. Verwendung einer Zusammensetzung, die in einem physiologisch akzeptablen Medium das Diosgenin oder einen Diosgenin-haltigen Dioscorea-Extrakt und Coffein enthält, um die Silhouette oder die Konturen des Gesichts zu verfeinern.

## Claims

1. Composition comprising, in a physiologically acceptable medium, diosgenin or a Dioscorea extract containing it, and caffeine.

2. Composition according to Claim 1, **characterized in that** the said Dioscorea extract is a wild yam rhizome extract.

3. Composition according to Claim 1 or 2, **characterized in that** caffeine represents from 0.01 to 10% of the total weight of the composition.

4. Composition according to Claim 3, **characterized in that** caffeine represents from 0.1 to 7% of the total weight of the composition.

5. Use of a composition comprising, in a physiologically acceptable medium, diosgenin or a Dioscorea extract containing it, and caffeine, for preventing or combating cellulite.

6. Use of a composition comprising, in a physiologically acceptable medium, diosgenin or a Dioscorea extract containing it, and caffeine, for trimming the figure or the contours of the face.
